# EUROPÄISCHE PATENTSCHRIFT

(11) **EP 3 300 649 B1**
(45) Veröffentlichungstag und Bekanntmachung des Hinweises auf die Patenterteilung: **23.01.2019**
(21) Anmeldenummer: 16191447.8
(22) Anmeldetag: 29.09.2016
(51) Int. Cl.: A47L 15/50

(54) **BELADUNGSTRÄGER FÜR EINEN REINIGUNGSAUTOMATEN**
LOAD PLATFORM FOR A CLEANING APPLIANCE
SUPPORT DE CHARGEMENT POUR UN APPAREIL DE NETTOYAGE

(43) Veröffentlichungstag der Anmeldung: 04.04.2018
(73) Patentinhaber: Belimed AG, 6300 Zug (CH)
(72) Erfinder: Fankhauser, Simon, 3257 Grossaffoltern (CH); Brun, Dominik, 6010 Kriens (CH); Jenko, Jan, 6005 Luzern (CH)
(74) Vertreter: Hepp Wenger Ryffel AG

(56) Entgegenhaltungen:
- EP-A1- 1 929 927
- EP-A2- 1 477 101
- DE-A1- 3 534 838
- DE-A1-102008 011 743

## Beschreibung

Die vorliegende Erfindung betrifft einen Beladungsträger für einen Reinigungsautomaten nach dem Oberbegriff von Anspruch 1.

Derartige Reinigungsautomaten werden zur Reinigung und zur Desinfektion von medizinischen Geräten verwendet. Um die Bestückung der einzelnen Beladungsträgeretagen mit sperrigen Gegenständen zu erlauben, sind die Beladungsträgeretagen oft aus dem Beladungsträger entfernbar. Die Beladungsträgeretagen sind zusätzlich mit einem drehbar gelagerten Wascharm versehen, welcher durch ein darin eingeleitetes Fluid in Rotation versetzbar ist, um die Verteilung des Fluids in einem Reinigungsraum des Reinigungsautomaten zu verbessern.

Die Beladungsträger der eingangs genannten Art können modular aufgebaut sein. Je nach Produkt können Beladungsträgeretagen und/oder Wascharme mit der dazugehörigen Zufuhrleitung weggelassen werden. So beschreibt die DE 10 2008 011 743 A1 einen flexibel konfigurierbaren Beladungsträger für einen Reinigungsautomaten.

Es kommt jedoch vor, dass Wascharme beim Aufbauen des Beladungsträgers vergessen werden. Die Reinigungsleistung entspricht dann nicht der geforderten Norm, so dass Chargen, wenn der Fehler überhaupt entdeckt wird, erneut gereinigt bzw. desinfiziert werden müssen. Eine solche Wiederholung ist zeit-, kosten- und energieintensiv.

Es ist eine Aufgabe der vorliegenden Erfindung, diese Nachteile im Stand der Technik zu überwinden. Insbesondere ist es eine Aufgabe der vorliegenden Erfindung, eine Vorrichtung der eingangs genannten Art zu schaffen, welche eine verbesserte Betriebssicherheit erlaubt und Fehlmanipulationen weitestgehend verhindert.

Die Aufgabe wird durch einen Beladungsträger mit den Merkmalen in Anspruch 1 gelöst. Der Beladungsträger für einen Reinigungsautomaten umfasst einen Tragrahmen mit einer unteren Beladungsträgeretage und einer oberen Beladungsträgeretage, welche parallel zueinander angeordnet und durch Abstandshalter miteinander verbunden sind. Ferner umfasst der Beladungsträger wenigstens eine weitere entfernbare Beladungsträgeretage, welche parallel zur oberen und unteren Beladungsträgeretage am Tragrahmen lösbar befestigt, bevorzugt in diesen einschiebbar, ist. Die entfernbare Beladungsträgeretage umfasst einen drehbar gelagerten Wascharm sowie wenigstens eine Zufuhrleitung zur Versorgung des Wascharmes mit einem Fluid.

Es sei an dieser Stelle vermerkt, dass die entfernbare Beladungsträgeretage nicht zwangsläufig zwischen der oberen und der unteren Beladungsträgeretage angeordnet sein muss. Die entfernbare Beladungsträgeretage kann beispielsweise oberhalb der oberen Beladungsträgeretage angeordnet sein.

Dadurch, dass die Beladungsträgeretage selbst einen Wascharm und wenigstens eine Zufuhrleitung zur Versorgung des Wascharmes mit einem Fluid umfasst, ist durch konstruktive Massnahmen ausgeschlossen, dass einer der Wascharme beim Aufbauen des Beladungsträgers vergessen wird, da Wascharm und Zufuhrleitung stets mit der entfernbaren Beladungsträgeretage, insbesondere gemeinsam (d.h in einem einzigen Arbeitsschritt), entfernt und wieder eingesetzt werden. Fehlmanipulationen während der Verwendung des Beladungsträgers werden damit im Wesentlichen vermieden. Eine stets konstante und den Normen entsprechende Reinigungsleistung ist gewährleistet.

Die Beladungsträgeretagen können eine im Wesentlichen rechteckige Form aufweisen, wobei die Abstandshalter an den Ecken der Beladungsträger und senkrecht zu diesen angeordnet sind. Da die Reinigungsräume der meisten Reinigungsautomaten eine rechteckige Grundfläche haben, wird durch diese Ausgestaltung der Beladungsträgeretagen eine optimale Raumausnutzung erzielt. Auch die genannte Anordnung der Abstandshalter erlaubt die Aufnahme einer möglichst grossen Anzahl von medizinischen Geräten auf einer Beladungsträgeretage.

Wenigstens ein Abstandshalter kann eine Fluidleitung umfassen. Dadurch kann Fluid von einer ersten Beladungsträgeretage zu einer zweiten gefördert werden. Die Fluidleitung kann mit Anschlussmitteln für die Zufuhrleitung der entfernbaren Beladungsträgeretage versehen sein.

Die entfernbare Beladungsträgeretage kann mit zwei Zufuhrleitungen versehen sein. Dadurch kann der Querschnitt der einzelnen Zufuhrleitung kleiner gewählt werden, was Platzeinsparungen bei den Anschlussmitteln für die Zufuhrleitung ermöglicht. Darüber hinaus ist bei Ausfall einer Zufuhrleitung noch immer eine Versorgung des Wascharmes mit Fluid gewährleistet.

Die Drehachse des Wascharmes kann durch den Schnittpunkt der Diagonalen der zugeordneten Beladungsträgeretage verlaufen. Dadurch wird eine ideale Verteilung des Fluids im Reinigungsraum des Reinigungsautomaten erzielt.

Insbesondere die obere Beladungsträgeretage kann einen drehbar gelagerten Wascharm und wenigstens eine Zufuhrleitung zur Versorgung des Wascharmes mit einem Fluid umfassen.

Ein Beladungsträger der oben genannten Art kann eine zwischen der unteren und der oberen Beladungsträgeretage fest angeordnete, mittlere Beladungsträgeretage umfassen, wobei jeweils eine entfernbare Beladungsträgeretage zwischen der unteren und der mittleren sowie zwischen der mittleren und der oberen Beladungsträgeretage angeordnet ist. Diese Ausgestaltung hat sich in der Praxis für die Reinigung und Desinfektion von häufig verwendeten medizinischen Geräten in Bezug auf ihre Grösse als vorteilhaft erwiesen.

Unterhalb der unteren Beladungsträgeretage kann ein Anschlussstutzen für die Versorgung des Wascharmes mit einem Fluid aus dem Reinigungsautomaten angeordnet sein.

Die Beladungsträgeretage kann an Schienen des Beladungsträgers verschiebbar gelagert sein. Dies erleichtert das Entfernen der Beladungsträgeretage. Die Schienen können als im Wesentlichen L-förmiger Profilvorsprung der darüber angeordneten Beladungsträgeretage ausgestaltet sein.

Die Erfindung wird nachfolgen anhand eines Ausführungsbeispiels in Verbindung mit der Zeichnung besser beschrieben.

Es zeigen schematisch:
- Fig. 1:: Eine perspektivische Ansicht eines erfindungsgemässen Beladungsträgers ohne Zwischenetagen;
- Fig. 2:: Eine perspektivische Ansicht einer Zwischenetage ohne Wascharm;

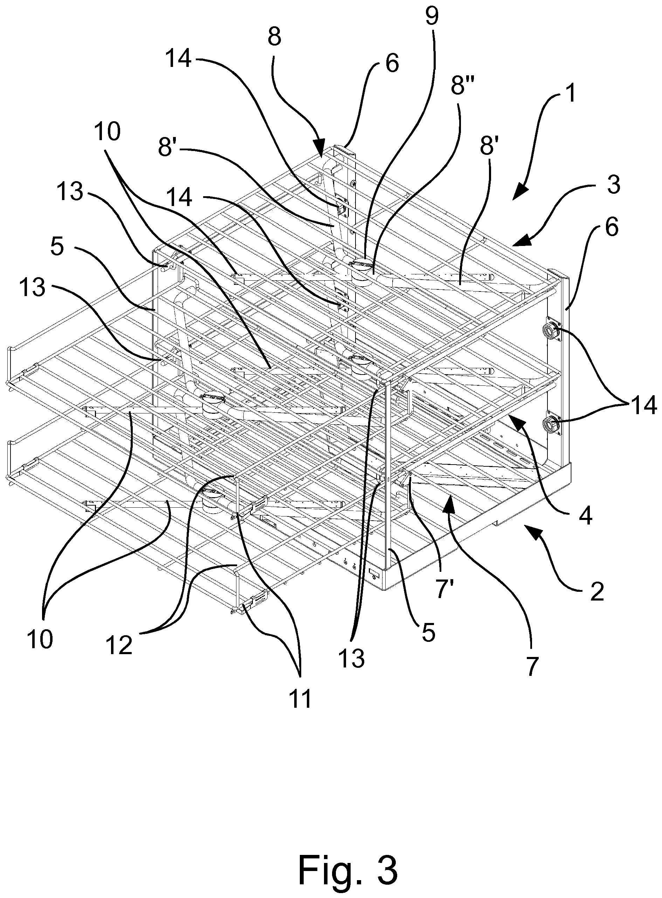

- Fig. 3:: Eine perspektivische Ansicht des Beladungsträgers der Figur 1 mit herausgezogenen Zwischenetagen; und
- Fig. 4:: Eine Detailansicht der Wascharmaufhängung;

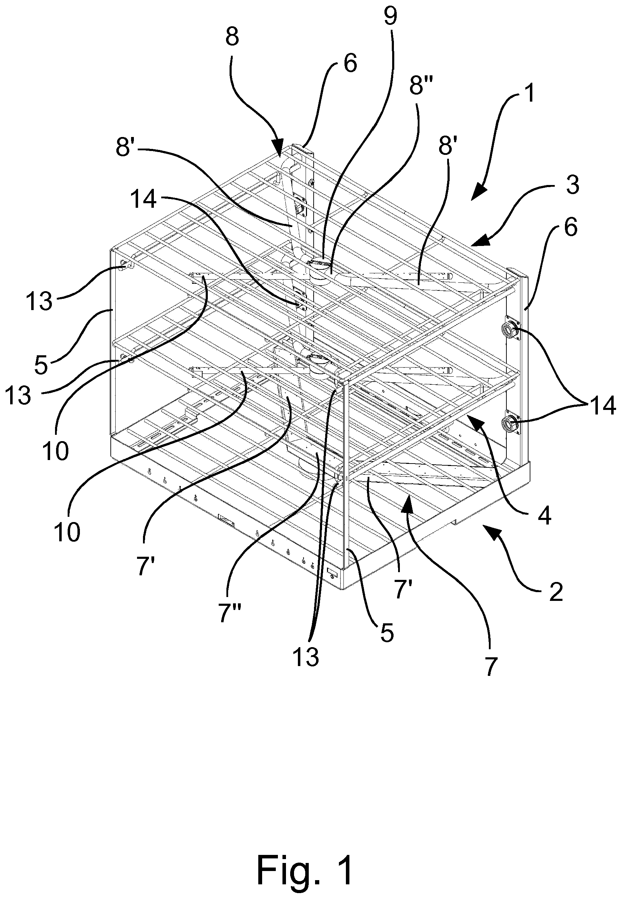

In der Figur 1 ist ein Beladungsträger 1 gezeigt. Dieser setzt sich aus drei Beladungsträgeretagen zusammen, nämlich einer unteren Beladungsträgeretage 2, einer oberen Beladungsträgeretage 3 und einer dazwischen angeordneten mittleren Beladungsträgeretage 4. Die Beladungsträgeretagen 2, 3 und 4 sind als Rost ausgebildet und parallel zueinander und voneinander gleich beabstandet angeordnet.

An den Ecken der rechteckigen Beladungsträgeretagen 2, 3 und 4 verlaufen Stützen 5 und 6, welche als Abstandshalter dienen und die Beladungsträgeretagen 2, 3 und 4 fest miteinander verbinden. Die Stützen 5 sind als einfacher Stab ausgebildet. Die Stützen 6 sind hohl ausgebildet und dienen der Zufuhr von Betriebsflüssigkeit.

Unterhalb der unteren Beladungsträgeretage ist eine Einspeisevorrichtung 7 für die Betriebsflüssigkeit angeordnet. Diese Einspeisevorrichtung 7 besteht aus zwei diagonal verlaufenden, hohlen Abschnitten 7', die mit der jeweiligen Stütze 6 fluidschlüssig verbunden sind. Im Bereich des Schnittpunktes der Diagonalen der unteren Beladungsträgeretage 2 ist ein mittlerer Abschnitt 7" angeordnet, welcher mit einem (nicht sichtbaren) Einlaufstutzen für die Betriebsflüssigkeit versehen ist. Wenn der Beladungsträger sich in einem Reinigungsautomaten befindet, kann über den mittleren Abschnitt 7" der Einspeisevorrichtung 7 eine fluidschlüssige Verbindung mit den diagonalen Abschnitten 7' und folglich mit den Stützen 6 hergestellt werden.

Die obere Beladungsträgeretage 3 und die mittlere Beladungsträgeretage 4 verfügen demgegenüber über eine Zulaufleitung 8, wobei der Übersicht halber nur die Zufuhrleitung 8 der oberen Beladungsträgeretage 3 mit Bezugszeichen versehen ist. Die Zulaufleitung 8 setzt sich ebenfalls aus zwei diagonal verlaufenden und an der jeweiligen Beladungsträgeretage aufgehängten Abschnitte 8' und einem im Bereich des Schnittpunktes der Diagonalen der jeweiligen Beladungsträgeretage angeordneten mittleren Abschnitt 8". Der Mittlere Abschnitt 8" umfasst ferner eine Aufhängevorrichtung 9 für einen Wascharm 10.

Der jeweilige Wascharm 10 einer Beladungsträgeretage kann somit über die Einspeisevorrichtung 7 und die hohlen Stützen 6 über die Zufuhrleitung 8 mit Betriebsflüssigkeit versorgt und in Rotation versetzt werden.

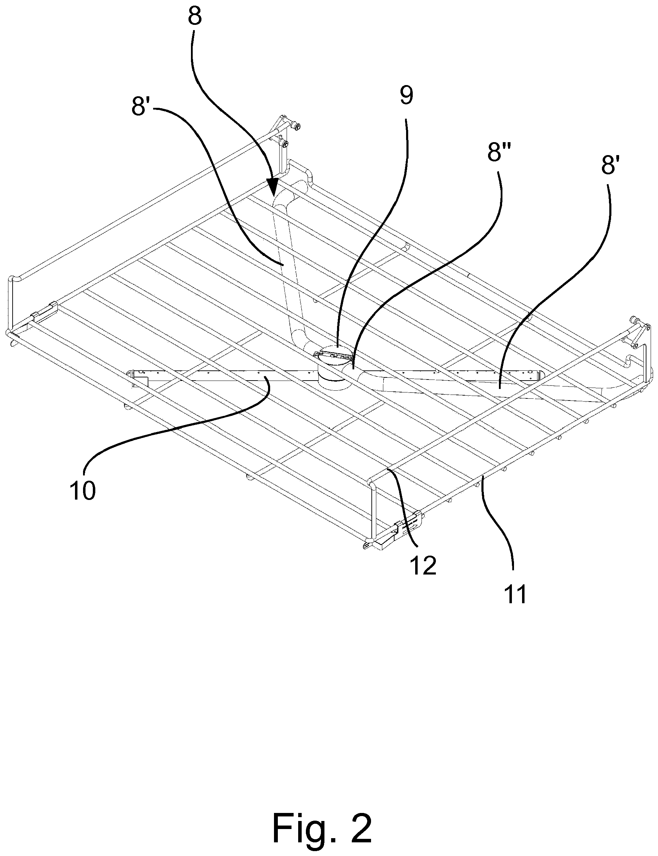

In der Figur 2 ist eine Zwischenetage 11 gezeigt. Die Zwischenetage 11 ähnelt im Aufbau der Beladungsträgeretagen 2, 3 und 4 und umfasst ebenfalls eine Zufuhrleitung 8, eine Aufhängevorrichtung 9 und einen Wascharm 10. Die Zwischenetage 11 umfasst jedoch an den kürzeren Kanten jeweils einen aus der Ebene der Zwischenetage 11 hervorstehenden Bügel 12.

Die obere Beladungsträgeretage 2 und die mittlere Beladungsträgeretage 3 weisen jeweils an ihrer Unterseite eine aufgehängte Schiene 13. Die Schienen 13 und die Bügel 12 sind komplementär zueinander ausgebildet, so dass die Zwischenetage 11 wie eine Schublade entlang der Schiene 13 verschiebbar angeordnet ist, so dass eine Entfernung der Zwischenetage 11 zur Beladung der unteren Beladungsträgeretage 2 und/oder der mittleren Beladungsträgeretage 4 mit sperrigen Gegenständen ermöglicht wird..

Bei einer Entnahme aus dem Beladungsträger 1 wird, wie in Figur 2 dargestellt, auch die Zufuhrleitung 8 und der Wascharm 10 entnommen. Ein Vergessen des Wascharmes 10 ist somit ausgeschlossen.

Da der Beladungsträger 1 mit oder ohne Zwischenetagen 11 verwendet werden kann, sind an den Stützen 6 Ventilanordnungen 14 vorgesehen, welche es einerseits ermöglichen, eine fluidschlüssige Verbindung zwischen der Stütze 6 und dem Wascharm 10 der Zwischenetage 11 aufzubauen und andererseits verhindern, dass die Betriebsflüssigkeit aus den Stützen 6 herausströmen kann, wenn keine Zwischenetage eingesetzt ist. Die Ventilanordnungen 14 sind nach Art eines Rückschlagventils oder als selbstverschliessende Klappe ausgebildet, so dass der in den Stützen 6 herrschende Fluiddruck die Ventilanordnung nach aussen hin abdichtet, wenn keine Zwischenetage eingesetzt ist und folglich keine Zufuhrleitung 8 in die Ventilanordnung 14 eingesteckt ist.

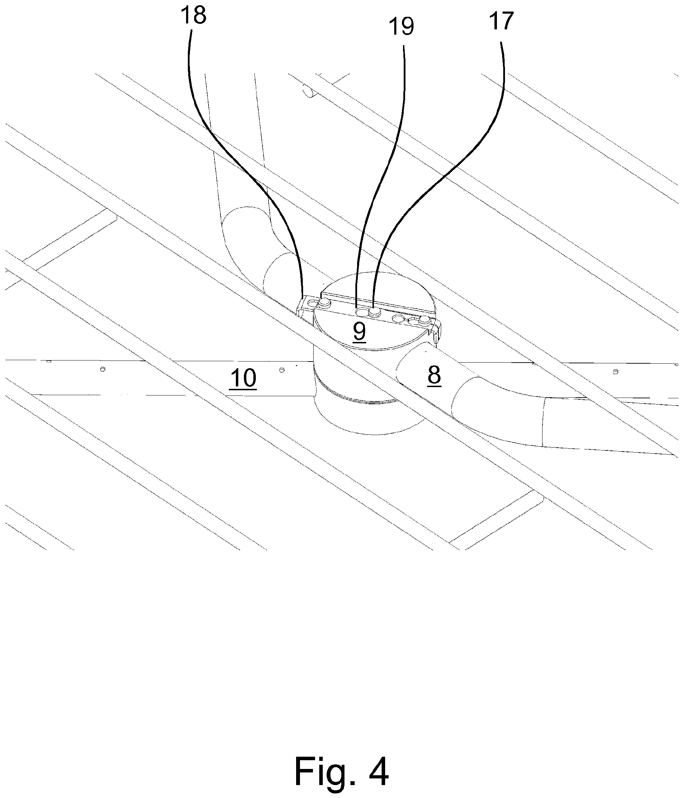

In der Figur 4 ist eine Detailansicht der Aufhängevorrichtung 9 für einen Wascharm 10 gezeigt. Der Wascharm ist über einen, durch die Aufhängevorrichtung 9 hindurchgehenden Bolzen 17 aufgehängt. An der Aufhängevorrichtung 9 ist ein Sicherungsschieber 18 vorhanden, welcher zwischen einer Freigabeposition und einer Schliessposition hin und her bewegbar ist. Der Sicherungsschieber weist eine Öffnung 19 mit einem schlüssellochähnlichen Profil auf. Mit dem Sicherungsschieber 18 in der Freigabeposition ist der Bolzen 17 aus der Aufhängevorrichtung 9 herausnehmbar, da die Öffnung 19 dem Bolzenquerschnitt entspricht. Mit dem Sicherungschieber 18 in der Schliessposition (und mit eingeschobenem Bolzen 17) wird jedoch der schmale Abschnitt der Öffnung 19 in eine Nut des Bolzens 17 gesteckt und der Wascharm 10 somit aufgehängt.

## Patentansprüche

1. Beladungsträger (1) für einen Reinigungsautomaten umfassend:
- Einen Tragrahmen mit einer unteren Beladungsträgeretage (2) und einer oberen Beladungsträgeretage (3), welche parallel zueinander angeordnet und durch Abstandshalter (5, 6) miteinander verbunden sind;
- Wenigstens eine weitere entfernbare Beladungsträgeretage (11), welche parallel zur oberen und unteren Beladungsträgeretage (2, 3) am Tragrahmen lösbar befestigt, bevorzugt in diesen einschiebbar, ist;
**dadurch gekennzeichnet, dass** die entfernbare Beladungsträgeretage (11) einen drehbar gelagerten Wascharm (10) sowie wenigstens eine Zufuhrleitung (8) zur Versorgung des Wascharmes (10) mit einem Fluid umfasst, insbesondere derart, dass der Wascharm (10) und die wenigstens eine Zufuhrleitung (8) mit der entfernbaren Beladungsträgeretage (11) gemeinsam entfernbar und wieder einsetzbar sind.

2. Beladungsträger (1) nach Anspruch 1, **dadurch gekennzeichnet, dass** die Beladungsträgeretagen (2, 3) eine im Wesentlichen rechteckige Form aufweisen, wobei die Abstandshalter (5, 6) an den Ecken der Beladungsträgeretagen (2, 3) und senkrecht zu diesen angeordnet sind.

3. Beladungsträger (1) nach Anspruch 1 oder 2, **dadurch gekennzeichnet, dass** wenigstens ein Abstandshalter (6) eine Fluidleitung umfasst.

4. Beladungsträger (1) nach Anspruch 3, **dadurch gekennzeichnet, dass** die Fluidleitung mit Anschlussmitteln (14) für die Zufuhrleitung (8) der entfernbaren Beladungsträgeretage (11) versehen ist.

5. Beladungsträger (1) nach Anspruch 4, **dadurch gekennzeichnet, dass** die entfernbare Beladungsträgeretage (11) mit zwei Zufuhrleitungen (8, 8') versehen ist.

6. Beladungsträger (1) nach einem der Ansprüche 2 bis 5, **dadurch gekennzeichnet, dass** die Drehachse des Wascharmes (10) durch den Schnittpunkt der Diagonalen der zugeordneten Beladungsträgeretage verläuft.

7. Beladungsträger (1) nach einem der vorhergehenden Ansprüche, **dadurch gekennzeichnet, dass** die obere Beladungsträgeretage (3) einen drehbar gelagerten Wascharm und wenigstens eine Zufuhrleitung (8') zur Versorgung des Wascharmes mit einem Fluid umfasst.

8. Beladungsträger (1) nach einem der vorhergehenden Ansprüche, **dadurch gekennzeichnet, dass** dieser eine zwischen der unteren und der oberen Beladungsträgeretage (2, 3) fest angeordnete, mittlere Beladungsträgeretage (4) umfasst, wobei jeweils eine entfernbare Beladungsträgeretage (11) zwischen der unteren und der mittleren (2, 4) sowie zwischen der mittleren und der oberen Beladungsträgeretage (3, 4) angeordnet ist.

9. Beladungsträger (1) nach einem der vorhergehenden Ansprüche, **dadurch gekennzeichnet, dass** unterhalb der unteren Beladungsträgeretage (2) ein Anschlussstutzen für die Versorgung des Wascharmes (10) mit einem Fluid aus dem Reinigungsautomaten angeordnet ist.

10. Beladungsträger (1) nach einem der vorhergehenden Ansprüche, **dadurch gekennzeichnet, dass** die entfernbare Beladungsträgeretage (11) an Schienen (13) des Beladungsträgers (1) verschiebbar gelagert ist.

11. Beladungsträger (1) nach Anspruch 10, **dadurch gekennzeichnet, dass** die Schienen (13) als im Wesentlichen L-förmiger Profilvorsprung der darüber angeordneten Beladungsträgeretage ausgestaltet sind.

## Claims

1. Load carrier (1) for a cleaning machine, comprising:
- a carrying frame with a lower load carrier tier (2) and an upper load carrier tier (3) which are arranged parallel to one another and which are connected to one another by spacers (5, 6);
- at least one further, removable load carrier tier (11) which, parallel to the upper and lower load carrier tiers (2, 3), is detachably fastened to, preferably slidable into, the carrying frame;
**characterized in that** the removable load carrier tier (11) comprises a rotatably mounted wash arm (10) and at least one feed line (8) for the supply of a fluid to the wash arm (10), in particular such that the wash arm (10) and the at least one feed line (8) are removable and re-insertable jointly with the removable load carrier tier (11).

2. Load carrier (1) according to Claim 1, **characterized in that** the load carrier tiers (2, 3) have a substantially rectangular shape, wherein the spacers (5, 6) are arranged at the corners of the load carrier tiers (2, 3) and perpendicular thereto.

3. Load carrier (1) according to Claim 1 or 2, **characterized in that** at least one spacer (6) comprises a fluid line.

4. Load carrier (1) according to Claim 3, **characterized in that** the fluid line is equipped with connector means (14) for the feed line (8) of the removable load carrier tier (11).

5. Load carrier (1) according to Claim 4, **characterized in that** the removable load carrier tier (11) is equipped with two feed lines (8, 8').

6. Load carrier (1) according to one of Claims 2 to 5, **characterized in that** the axis of rotation of the wash arm (10) runs through the intersection point of the diagonals of the associated load carrier tier.

7. Load carrier (1) according to one of the preceding claims, **characterized in that** the upper load carrier tier (3) comprises a rotatably mounted wash arm and at least one feed line (8') for the supply of a fluid to the wash arm.

8. Load carrier (1) according to one of the preceding claims, **characterized in that** said load carrier comprises a middle load carrier tier (4) which is arranged fixedly between the lower and the upper load carrier tiers (2, 3), wherein in each case one removable load carrier tier (11) is arranged between the lower and the middle (2, 4) and between the middle and the upper load carrier tiers (3, 4).

9. Load carrier (1) according to one of the preceding claims, **characterized in that** a connector piece for the supply of a fluid from the cleaning machine to the wash arm (10) is arranged below the lower load carrier tier (2).

10. Load carrier (1) according to one of the preceding claims, **characterized in that** the removable load carrier tier (11) is mounted displaceably on rails (13) of the load carrier (1) .

11. Load carrier (1) according to Claim 10, **characterized in that** the rails (13) are designed as substantially L-shaped profile projections of the load carrier tier arranged thereabove.

## Revendications

1. Support de chargement (1) pour un automate de nettoyage, comprenant :
- un cadre porteur avec un étage de support de chargement inférieur (2) et un étage de support de chargement supérieur (3), qui sont disposés parallèlement l'un à l'autre et qui sont connectés l'un à l'autre par des éléments d'espacement (5, 6) ;
- au moins un étage de support de chargement amovible supplémentaire (11) qui est fixé de manière amovible au cadre porteur parallèlement aux étages de support de chargement supérieur et inférieur (2, 3), de préférence qui peut être inséré dans celui-ci ;
**caractérisé en ce que** l'étage de support de chargement amovible (11) comprend un bras de lavage (10) supporté à rotation ainsi qu'au moins une conduite d'alimentation (8) pour alimenter en fluide le bras de lavage (10), en particulier de telle sorte que le bras de lavage (10) et l'au moins une conduite d'alimentation (8) puissent être enlevés et installés conjointement avec l'étage de support de chargement amovible (11).

2. Support de chargement (1) selon la revendication 1, **caractérisé en ce que** les étages de support de chargement (2, 3) présentent une forme sensiblement rectangulaire, les éléments d'espacement (5, 6) étant disposés au niveau des coins des étages de support de chargement (2, 3) et étant perpendiculaires à ceux-ci.

3. Support de chargement (1) selon la revendication 1 ou 2, **caractérisé en ce qu'**au moins un élément d'espacement (6) comprend une conduite de fluide.

4. Support de chargement (1) selon la revendication 3, **caractérisé en ce que** la conduite de fluide est pourvue de moyens de raccordement (14) pour la conduite d'alimentation (8) de l'étage de support de chargement amovible (11).

5. Support de chargement (1) selon la revendication 4, **caractérisé en ce que** l'étage de support de chargement amovible (11) est pourvu de deux conduites d'alimentation (8, 8').

6. Support de chargement (1) selon l'une quelconque des revendications 2 à 5, **caractérisé en ce que** l'axe de rotation du bras de lavage (10) s'étend à travers le point d'intersection des diagonales des étages de support de chargement associés.

7. Support de chargement (1) selon l'une quelconque des revendications précédentes, **caractérisé en ce que** l'étage de support de chargement supérieur (3) comprend un bras de lavage supporté à rotation et au moins une conduite d'alimentation (8') pour l'alimentation en fluide du bras de lavage.

8. Support de chargement (1) selon l'une quelconque des revendications précédentes, **caractérisé en ce que** celui-ci comprend un étage de support de chargement central (4) disposé fixement entre les étages de support de chargement inférieur et supérieur (2, 3), un étage de support de chargement amovible (11) étant à chaque fois disposé entre les étages de support de chargement inférieur et central (2, 4) et entre les étages de support de chargement central et supérieur (3, 4).

9. Support de chargement (1) selon l'une quelconque des revendications précédentes, **caractérisé en ce qu'**en dessous de l'étage de support de chargement inférieur (2) est disposée une tubulure de raccordement pour l'alimentation en fluide du bras de lavage (10) à partir de l'automate de nettoyage.

10. Support de chargement (1) selon l'une quelconque des revendications précédentes, **caractérisé en ce que** l'étage de support de chargement amovible (11) est supporté de manière déplaçable sur des rails (13) du support de chargement (1).

11. Support de chargement (1) selon la revendication 10, **caractérisé en ce que** les rails (13) sont configurés sous forme de saillie profilée essentiellement en forme de L de l'étage de support de chargement disposé au-dessus.
